# EUROPEAN PATENT APPLICATION

(11) **EP 4 194 456 A1**
(43) Date of publication of application: **14.06.2023**
(21) Application number: 21213131.2
(22) Date of filing: 08.12.2021
(51) Int. Cl.: C07D 471/06, A61P 25/28, C07D 311/92, C07D 471/16, C07D 491/16, C07D 493/16, C07F 9/48, C07F 9/6571

(54) **FUSED TRICYCLIC COMPOUNDS USEFUL IN THE TREATMENT OF NEURODEGENERATIVE DISEASES**

(71) Applicant: Julius-Maximilians-Universität Würzburg, 97070 Würzburg (DE)
(72) Inventor: Dandekar, Thomas, 97074 Würzburg (DE); Sarukhanyan, Edita, 97070 Würzburg (DE)
(74) Representative: Schiweck Weinzierl Koch Patentanwälte Partnerschaft mbB

(57) **Abstract**

The present invention relates to a new compound according to formula (I) as well as to the use of this compound in medicine, in the prevention of the aggregation of phosphorylated Tau proteins, the treatment or prevention of a tauopathy and in the treatment or prevention of a neurodegenerative disease, preferably selected from the group consisting of Primary age-related tauopathy (PART) dementia, Chronic traumatic encephalopathy, Progressive supranuclear palsy, Corticobasal degeneration and Alzheimer's disease, more preferably Alzheimer's disease.

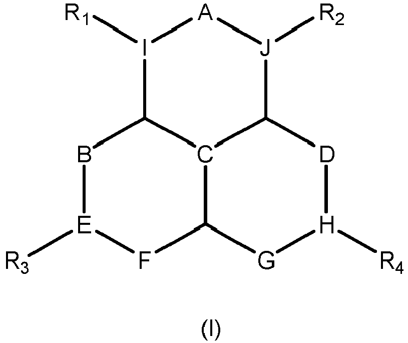

## Description

### TECHNICAL FIELD OF THE INVENTION

The present invention relates to a new compound according to formula (I) as well as to the use of this compound in medicine, in the prevention of the aggregation of phosphorylated Tau proteins, the treatment or prevention of a tauopathy and in the treatment or prevention of a neurodegenerative disease, preferably selected from the group consisting of Primary age-related tauopathy (PART) dementia, Chronic traumatic encephalopathy, Progressive supranuclear palsy, Corticobasal degeneration and Alzheimer's disease, more preferably Alzheimer's disease.

### BACKGROUND ART

Microtubules (MT) are hollow tubes formed by the assembly of α and β tubulin dimers¹. Microtubule mobility in neurons is maintained by several proteins, the so-called microtubule-associated proteins ².

A protein that plays an important role in MT stabilization and promotes neurite outgrowth is the protein Tau . The Tau protein in human brain is encoded by 11 exons, which are subsequently spliced to form six isoforms with the range of 352-441 amino acids³. The six isoforms of Tau differ either in presence or absence of one or two inserts (N1 and N2) at the N-terminal part and three or four repeats (R1, R2, R3 and R4) at the C-terminal region². The longest isoform of Tau consists of four repeats and two inserts achieving a length of 441 amino acids, while the shortest isoform has only three repeats (R1, R3 and R4) and no insert, resulting in a length of 352 amino acids in total⁴.

Binding of Tau proteins to MT is facilitated through the MT binding domains. However, MT assembly strongly depends on the phosphorylation state of Tau proteins: The more phosphorylated a Tau protein is, the weaker is its effect on MT polymerization^{3,5,6,} *In vitro* tests show that the phosphorylation of Ser 262 significantly reduces the affinity of Tau to MTs ⁵.

Neurodegenerative diseases, such as Alzheimer's disease (AD), develop when Tau is mutated or abnormally phosphorylated^{1,7}. In this case, the protein loses its affinity towards MTs and aggregates into neurofibrillary tangles^{1 ,8 ,} . Further diseases wherein mutated or abnormally phosphorylated Tau plays as role are Primary age-related tauopathy (PART) dementia, Chronic traumatic encephalopathy, Progressive supranuclear palsy, and Corticobasal degeneration.

Thus, the compounds that would bind to phosphorylated Tau proteins and prevent them from both detachment and aggregation could be an optimal solution for microtubule stabilization². The first compound found to promote the polymerization of tubulin heterodimers into microtubules was the anti-cancer drug paclitaxel^{9,} . At their discovery by Horwitz and coworkers in 1978⁹ paclitaxel and its analogs were considered the only class of compounds acting as microtubule stabilizers. However, since 1995 the discovery of new compounds, which have, similar to taxanes, the ability to fix depolymerization of microtubules, opened a new opportunity for the next generation of anti-cancer agents. These include epothilones A and B⁹, discodermolide⁹, eleutherobin⁹, sarcodictyines A and B ⁹laulimalide⁹ and FR182877⁹. However, the Tau stabilizers found and tested so far failed to reach the clinic because of toxic side effects (paclitaxel), or were removed from the application for AD (epotilone D), or are in Phase I clinical trials (TPI 287) for mild to moderate AD¹⁰.

Another therapeutic approach to tackle AD is the prevention of aggregation regardless of phosphorylation or other Tau modification. Derivatives of methylene blue revealed the ability to disrupt the aggregation of Tau, which entailed some improvement in AD-related symptoms, but failed in Phase II due to the side effects such as diarrhea, urinary urgency, painful urination and dizziness¹⁰.

Thus, there is a need for further compounds useful in the theraphy of tauopathies such as Alzheimer's disease.

### SUMMARY OF THE INVENTION

The invention relates to a compound according to formula (I) 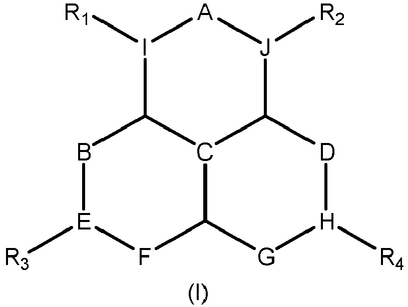 wherein
R₁ is selected from the group consisting of a) absent if I is CH₂ or 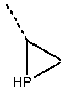 b) halogen, -OH, -NH₂, -N=S, -P(OH)₂, -PH(OH), and -COOH if I is CH;
R₂ is selected from the group consisting of a) absent if J is CH₂ or
   b) halogen, -OH, -NH₂, -N=S, -P(OH)₂, -PH(OH), 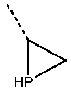 and -COOH if J is CH;
R₃ is selected from the group consisting of a) absent if E is CH₂; or
   b) halogen, and -COOH , -PH(OH), -P(OH)₂, -OH, - NH₂, -CH(OH)₂, -N=O if E is CH; or
   c) =O if E is C;
R₄ is selected from the group consisting of a) absent if H is CH₂; or
   b) halogen, -COOH, -CH(OH)₂, -PH(OH), -P(OH)₂, - CH(OH)₂, -OH, -NH₂, and -N=O if H is CH; or
   c) =O if H is C;
A is selected from the group consisting of CH₂, O, and C=O;
B is selected from the group consisting of CH₂, O, and -NH-;
C is selected from the group consisting of CH, and N,
D is selected from the group consisting of CH₂, O, and -NH-;
E is selected from the group consisting of CH₂, CH, and C;
F is selected from the group consisting of NH, and CH₂;
G is selected from the group consisting of NH, and CH₂;
H is selected from the group consisting of CH₂, CH, and C;
I is selected from the group consisting of CH or CH₂;
J is selected from the group consisting of CH or CH₂ or
or a pharmaceutically acceptable salt, solvate or hydrate thereof.

In a further aspect, the compound of the present invention is for use in medicine.

In a further aspect, the invention relates to the compound for use in the prevention of the aggregation of phosphorylated Tau proteins.

In a further aspect, the invention relates to the compound for use in the treatment or prevention of a tauopathy.

In a further aspect, the invention relates to the compound for use in the treatment or prevention of a neudegenerative disease, preferferably selected from the group consisting of Primary age-related tauopathy (PART) dementia, Chronic traumatic encephalopathy, Progressive supranuclear palsy, Corticobasal degeneration as well as other neurodegenerative disease and Alzheimer's disease, more preferably Alzheimer's disease.

As demonstrated in the examples, the compounds of the present invention allow prevention of aggregation of phosphorylated Tau proteins and thus the prevention of tauopathies. Moreover, the examples show, that the compounds of the present invention achieve also tau de-bundling and therefore are useful in the treatment of patients who already suffer from a tauopathy such as Alheimer's disease.

### BRIEF DESCRIPTION OF THE FIGURES

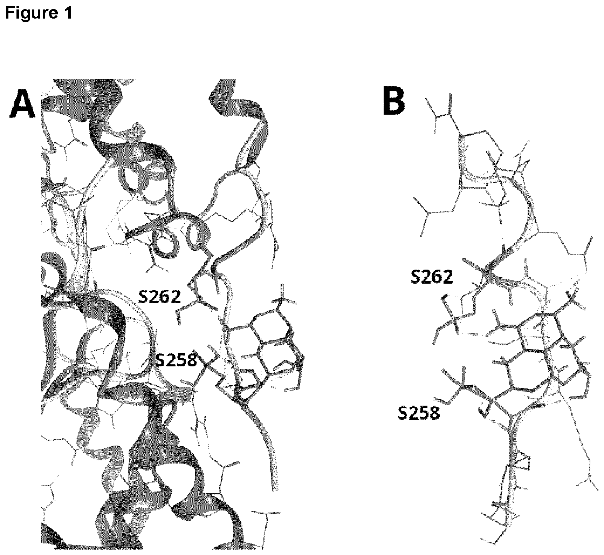

**Fig. 1****: Compound 10 (A and B) shown in a complex with MT-associated Tau peptide and single Tau.** Results from molecular docking simulation. The compounds are highlighted in green (compound 10), the protein is shown as a secondary structure, the key residues - phosphorylated S262 and S258 are highlighted in yellow, phosphorus atom is shown in magenta. H-bonds between the compound and serine residues are shown in light blue dashed lines.

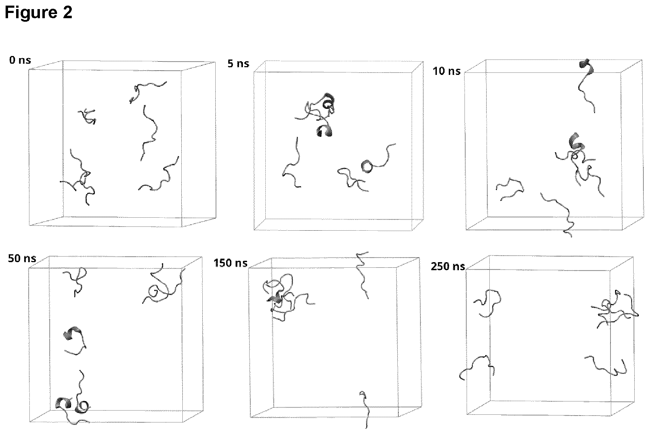

**Fig. 2****: Dynamics of native Tau peptides over time.** The peptides are shown as a secondary structure representation and highlighted in blue. The cubic simulation box of size 7×7×7 nm is shown at different angles to achieve the best view perspective. Water molecules and ions are omitted for clarity.

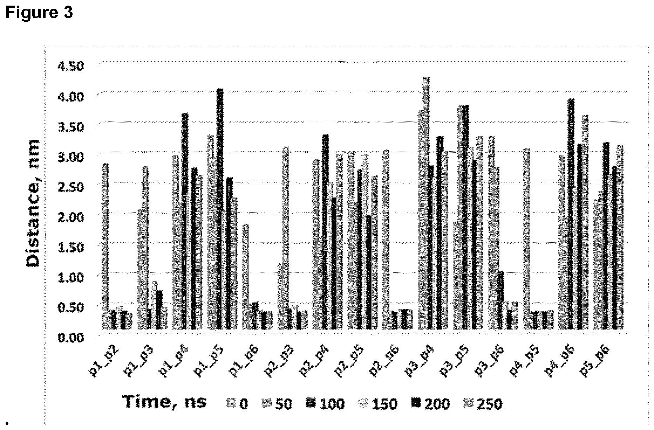

**Fig. 3****: Minimum distances vs. time frame between the backbones of Tau peptide pairs shown as 3D bars.** Color code refers to different time frames - 0, 50, 100, 150, 200 and 250 ns, the height of the bars corresponds to the distances in nm between the backbones of p1_p2, p1_p3, p1_p4, etc. peptide pairs at particular time frame.

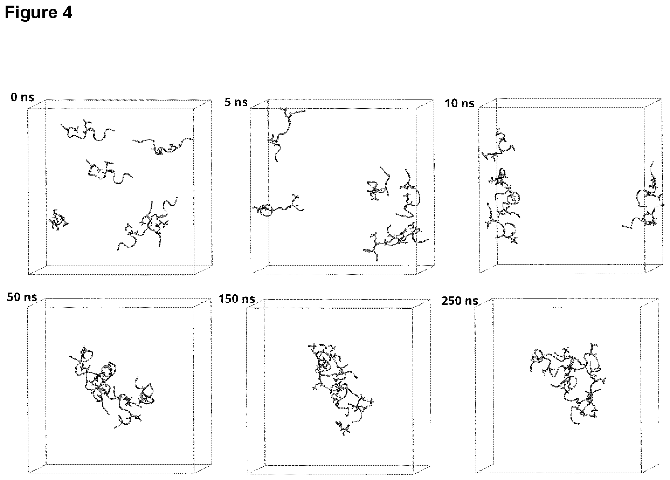

**Fig. 4****: Dynamics of phosphorylated Tau peptides at different time frames (0, 5, 10, 50, 150, 250 ns).** The peptides are shown as a secondary structure representation and highlighted in green. The phosphorylated serine residues (Ser 258 and Ser 262) are shown as a licorice and highlighted in magenta. The cubic simulation box of size 7 × 7 × 7 nm is shown at different angles to achieve the best view perspective. Water molecules and ions are omitted for clarity.

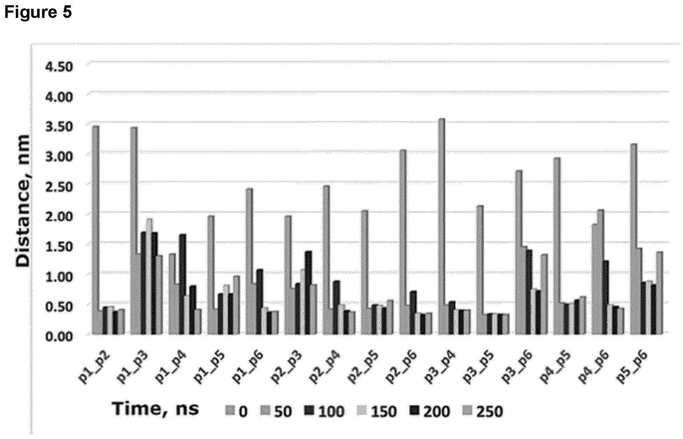

**Fig. 5****: Minimum distances vs. time frame between the backbones of phosphorylated Tau peptide pairs shown as 3D bars.** Color code refers to different time frames - 0, 50, 100, 150, 200 and 250 ns, the height of the bars corresponds to the distances in nm between the backbones of p1_p2, p1_p3, p1_p4, etc. peptide pairs at particular time frame.

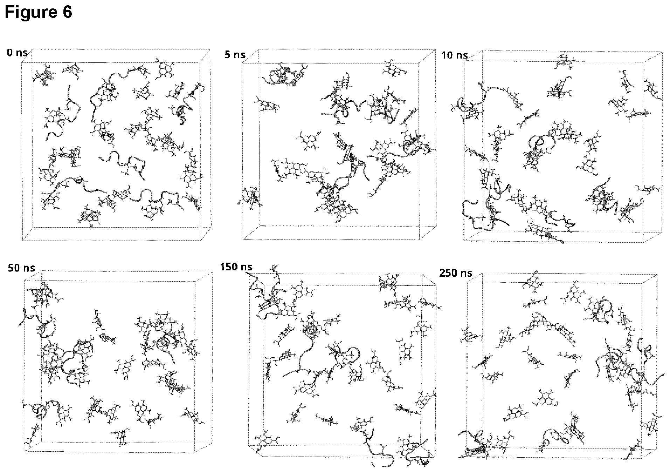

**Fig. 6****: Dynamics of phosphorylated Tau peptides in the presence of compound 10 and water at different time frames (T=300K).** The peptides are shown as a secondary structure representation and highlighted in green. The phosphorylated serine residues (Ser 258 and Ser 262) are shown as a licorice and highlighted in magenta. The compounds are represented in licorice and highlighted in violet. The cubic simulation box of size 7 × 7 × 7 nm is shown at different angles to achieve the best viewing perspective. Water molecules and ions are omitted for clarity.

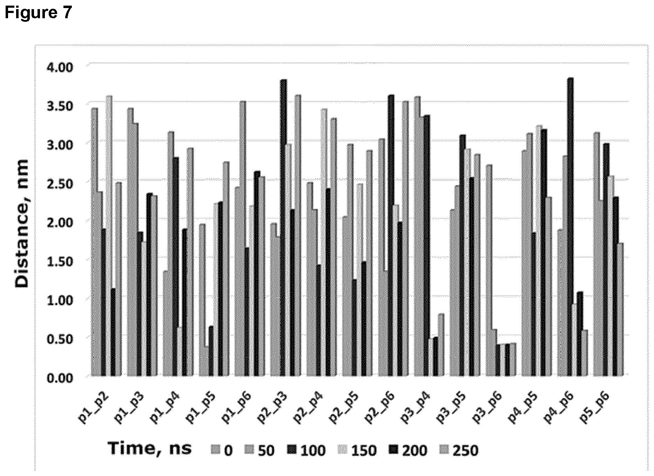

**Fig. 7**: 3D bars representing minimum distances vs. time frame between the **backbones of phosphorylated Tau peptide pairs under the influence of compounds 10.** Color code refers to different time frames - 0, 50, 100, 200 and 250 ns, the height of the bars corresponds to the distances in nm between the backbones of p1_p2, p1_p3, p1_p4, etc. peptide pairs at particular time frame.

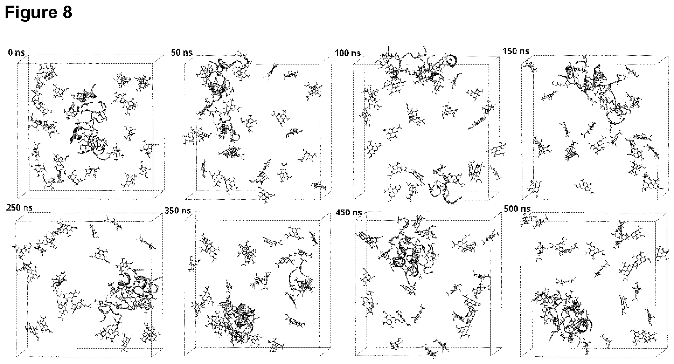

**Fig. 8****: Dynamics of phosphorylated Tau bundle in the presence of low concentration of compound 10 and water at different time frames.** The peptides are shown as a secondary structure representation and highlighted in green. The phosphorylated serine residues (Ser 258 and Ser 262) are shown as a licorice and highlighted in magenta. The phosphorus atom is shown in yellow as a van der Waals bead. The compounds are represented in licorice and highlighted in violet. The cubic simulation box of size 7 × 7 × 7 nm is shown at different angles to achieve the best viewing perspective. Water molecules and ions are omitted for clarity.

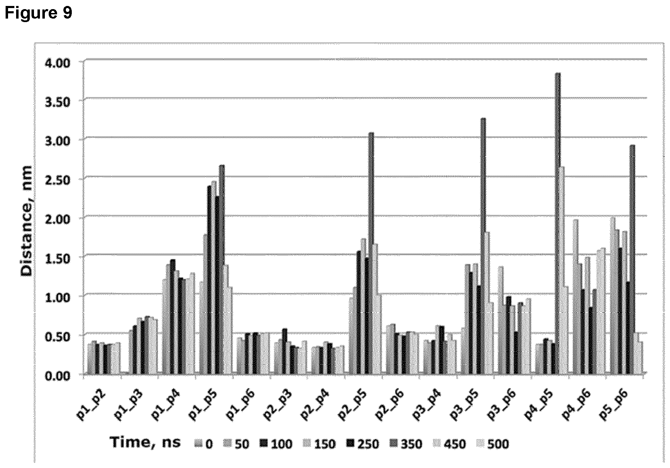

**Fig. 9**: 3D bars representing minimum distances vs. time frame between the **backbones of phosphorylated Tau peptide pairs in a bundle under the influence of compounds 10 at low concentration.** Color code refers to different time frames - 0, 50, 100, 150, 250, 350, 450 and 500 ns, the height of the bars corresponds to the distances in nm between the backbones of p1_p2, p1_p3, p1_p4, etc. peptide pairs at particular time frame.

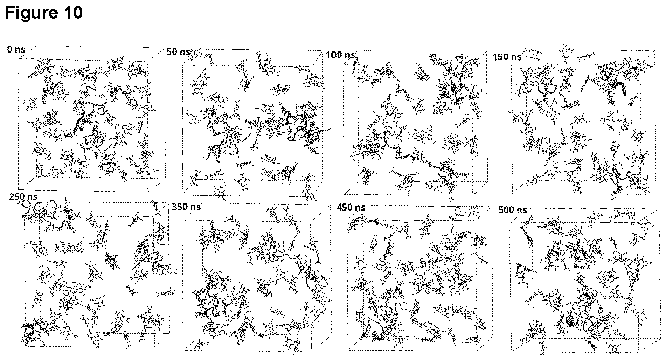

**Fig. 10****: Dynamics of phosphorylated tau bundle in the presence of high concentration of compound 10 and water at different time frames.** The peptides are shown as a secondary structure representation and highlighted in green. The phosphorylated serine residues (Ser 258 and Ser 262) are shown as a licorice and highlighted in magenta. The phosphorus atom is shown in yellow as a van der Waals bead. The compounds are represented in licorice and highlighted in violet. The cubic simulation box of size 7 × 7 × 7 nm is shown at different angles to achieve the best viewing perspective. Water molecules and ions are omitted for clarity.

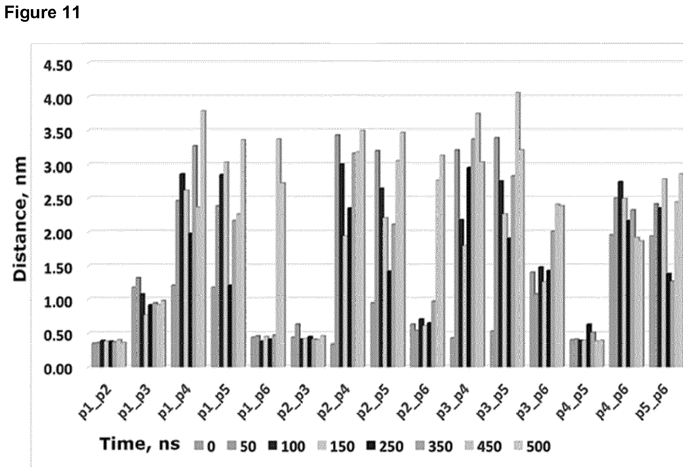

**Fig. 11**: 3D bars representing minimum distances vs. time frame between the **backbones of phosphorylated Tau peptide pairs in a bundle under the influence of compound 10 at high concentration.** Color code refers to different time frames - 0, 50, 100, 150, 250, 350, 450 and 500 ns, the height of the bars corresponds to the distances in nm between the backbones of p1_p2, p1_p3, p1_p4, etc. peptide pairs at particular time frame.

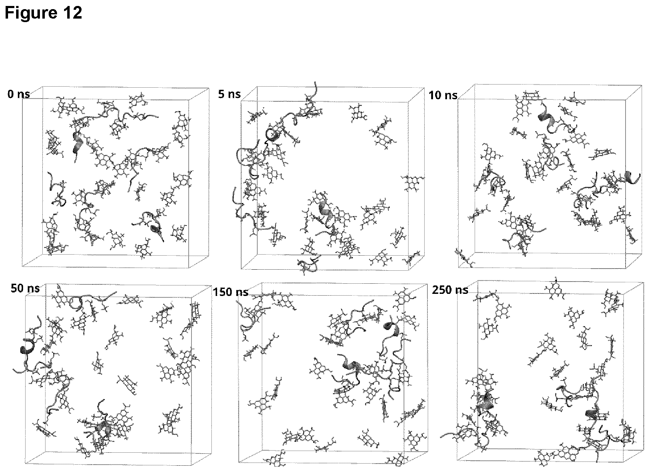

**Fig. 12****: Dynamics of phosphorylated Tau peptides in the presence of compound 10 and water at different time frames (T=310K).** The peptides are shown as a secondary structure representation and highlighted in green. The phosphorylated serine residues (Ser 258 and Ser 262) are shown as a licorice and highlighted in magenta. The compounds are represented in licorice and highlighted in violet. The cubic simulation box of size 7 × 7 × 7 nm is shown at different angles to achieve the best viewing perspective. Water molecules and ions are omitted for clarity.

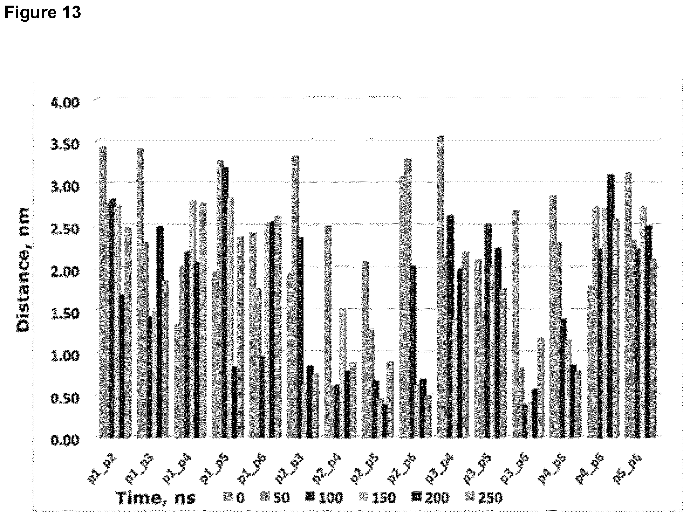

**Fig. 13**: 3D bars representing minimum distances vs. time frame between the **backbones of phosphorylated Tau peptide pairs under the influence of compound 10 (T=310K).** Color code refers to different time frames - 0, 50, 100, 200 and 250 ns, the height of the bars corresponds to the distances in nm between the backbones of p1_p2, p1_p3, p1_p4, etc. peptide pairs at particular time frame.

### DETAILED DESCRIPTION OF THE INVENTION

### Definitions

The term "halogen" or "halo" means fluoro, chloro, bromo, or iodo.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

As used herein and throughout the entire description, the term "solvate" as used herein refers to an addition complex of a dissolved material in a solvent (such as an organic solvent (e.g., an aliphatic alcohol (such as methanol, ethanol, n-propanol, isopropanol), acetone, acetonitrile, ether, and the like), water or a mixture of two or more of these liquids), wherein the addition complex exists in the form of a crystal or mixed crystal. The amount of solvent contained in the addition complex may be stoichiometric or non-stoichiometric. A "hydrate" is a solvate wherein the solvent is water.

As used herein and throughout the entire description, the term "pharmaceutically acceptable salt" refers to a salt that retains the desired biological activity of the parent compound and does not impart any undesired toxicological effects (see e.g., Berge, S. M., et al. (1977) J. Pharm. Sci. 66: 1-19). Physiologically acceptable salts of the compounds of the present invention are in particular salts with a nontoxic salt component and preferably are pharmaceutically utilizable salts. They can contain inorganic or organic salt components. Such salts can be formed, for example, from compounds of the present invention which contain an acidic group, for example a carboxylic acid group (HO-CO-) or a sulfonic acid group (HO-S(O)₂-) and nontoxic inorganic or organic bases. Suitable bases are, for example, alkali metal compounds or alkaline earth metal compounds, such as sodium hydroxide, potassium hydroxide, sodium carbonate or sodium hydrogencarbonate, or ammonia, organic amino compounds and quaternary ammonium hydroxides. Reactions of compounds of the present invention with bases for the preparation of the salts are in general carried out according to customary procedures in a solvent or diluent. On account of the physiological and chemical stability, advantageous salts of acidic groups are in many cases sodium, potassium, magnesium or calcium salts or ammonium salts which can also carry one or more organic groups on the nitrogen atom. Compounds of the present invention which contain a basic, i.e. protonatable, group, for example an amino group or another basic heterocycle, can be present in the form of their acid addition salts with physiologically acceptable acids, for example as salt with hydrogen chloride, hydrogen bromide, phosphoric acid, sulfuric acid, acetic acid, benzoic acid, methanesulfonic acid, p-toluenesulfonic acid, which in general can be prepared from the compounds of the present invention by reaction with an acid in a solvent or diluent according to customary procedures. As usual, in particular in the case of acid addition salts of a compound containing two or more basic groups, in an obtained salt the ratio of the salt components can deviate upward or downward from the stoichiometric ratio, such as the molar ratio 1:1 or 1:2 in the case of the acid addition salt of a compound of the present invention containing one or two basic groups with a monovalent acid, and vary depending on the applied conditions. The present invention comprises also salts containing the components in a non-stoichiometric ratio, and an indication that an acid addition salt of a compound of the present invention contains an acid in equimolar amount, for example, also allows for a lower or higher amount of acid in the obtained salt, for example about 0.8 or about 1.1 mol of acid per mol of compound of the present invention. If the compounds of the present invention simultaneously contain an acidic and a basic group in the molecule, the invention also includes internal salts (betaines, zwitterions) in addition to the salt forms mentioned. The present invention also comprises all salts of the compounds of the present invention which, because of low physiological tolerability, are not directly suitable for use as a pharmaceutical, but are suitable as intermediates for chemical reactions or for the preparation of physiologically acceptable salts, for example by means of anion exchange or cation exchange. A subject of the present invention also are solvates of the compounds of the present invention and their salts, such as hydrates and adducts with alcohols like (C₁-C₄)-alkanols, in particular physiologically acceptable solvates, as well as active metabolites of compounds of the present invention.

### Compounds

The invention is directed to a compound according to formula (I) 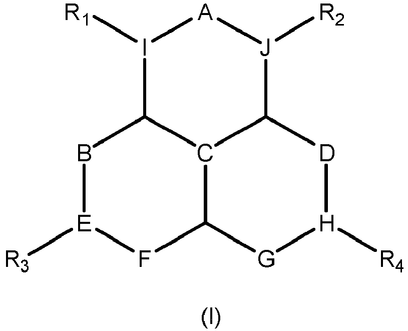 wherein
R₁ is selected from the group consisting of a) absent if I is CH₂ or
   b) halogen, -OH, -NH₂, -N=S, -P(OH)₂, -PH(OH), 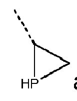 and -COOH if I is CH.
R₂ is selected from the group consisting of a) absent if J is CH₂ or
   b) halogen, -OH, -NH₂, -N=S, -P(OH)₂, -PH(OH), 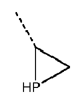 and -COOH if J is CH.
R₃ is selected from the group consisting of a) absent if E is CH₂; or
   b) halogen, and -COOH , -PH(OH), -P(OH)₂, -OH, - NH₂, -CH(OH)₂, -N=O if E is CH; or
   c) =O if E is C.
R₄ is selected from the group consisting of a) absent if H is CH₂; or
   b) halogen, -COOH, -CH(OH)₂, -PH(OH), -P(OH)₂, - CH(OH)₂, -OH, -NH₂, and -N=O if H is CH; or
   c) =O if H is C.
A is selected from the group consisting of CH₂, O, and C=O.
B is selected from the group consisting of CH₂, O, and -NH-.
C is selected from the group consisting of CH, and N.
D is selected from the group consisting of CH₂, O, and -NH-.
E is selected from the group consisting of CH₂, CH, and C.
F is selected from the group consisting of NH, and CH₂.
G is selected from the group consisting of NH, and CH₂.
H is selected from the group consisting of CH₂, CH, and C.
Wherein "H", here, similar to A, B, C etc. is a variable rather than the chemical symbol for hydrogen. Thus, it can have more than one bond. In contrast, in specific chemical radicals like "CH₂", "CH", "NH" etc. "H" symbolizes hydrogen and therefore has only one bond.
I is selected from the group consisting of CH or CH₂;
J is selected from the group consisting of CH or CH₂;
or a pharmaceutically acceptable salt, solvate or hydrate thereof.

The halogen is selected from Cl, Br, I, F preferably F.

In one embodiment,
R₁ is selected from the group consisting of -NH₂, -P(OH)₂, preferably -NH₂;
R₂ is selected from the group consisting of -NH₂, -P(OH)₂, preferably -NH₂;
R₃ is selected from the group consisting of -CH(OH)₂, -P(OH)₂, preferably -CH(OH)₂;
R₄ is selected from the group consisting of -P(OH)₂, -CH(OH)₂, preferably -CH(OH)₂;
A is O;
B is selected from the group consisting of CH₂, and -NH-;
C is CH;
D is selected from the group consisting of CH₂, and -NH-;
E is CH;
F is CH₂;
G is CH₂;
H is CH;
I is selected from the group consisting of CH;
J is selected from the group consisting of CH;
or a pharmaceutically acceptable salt, solvate or hydrate thereof.

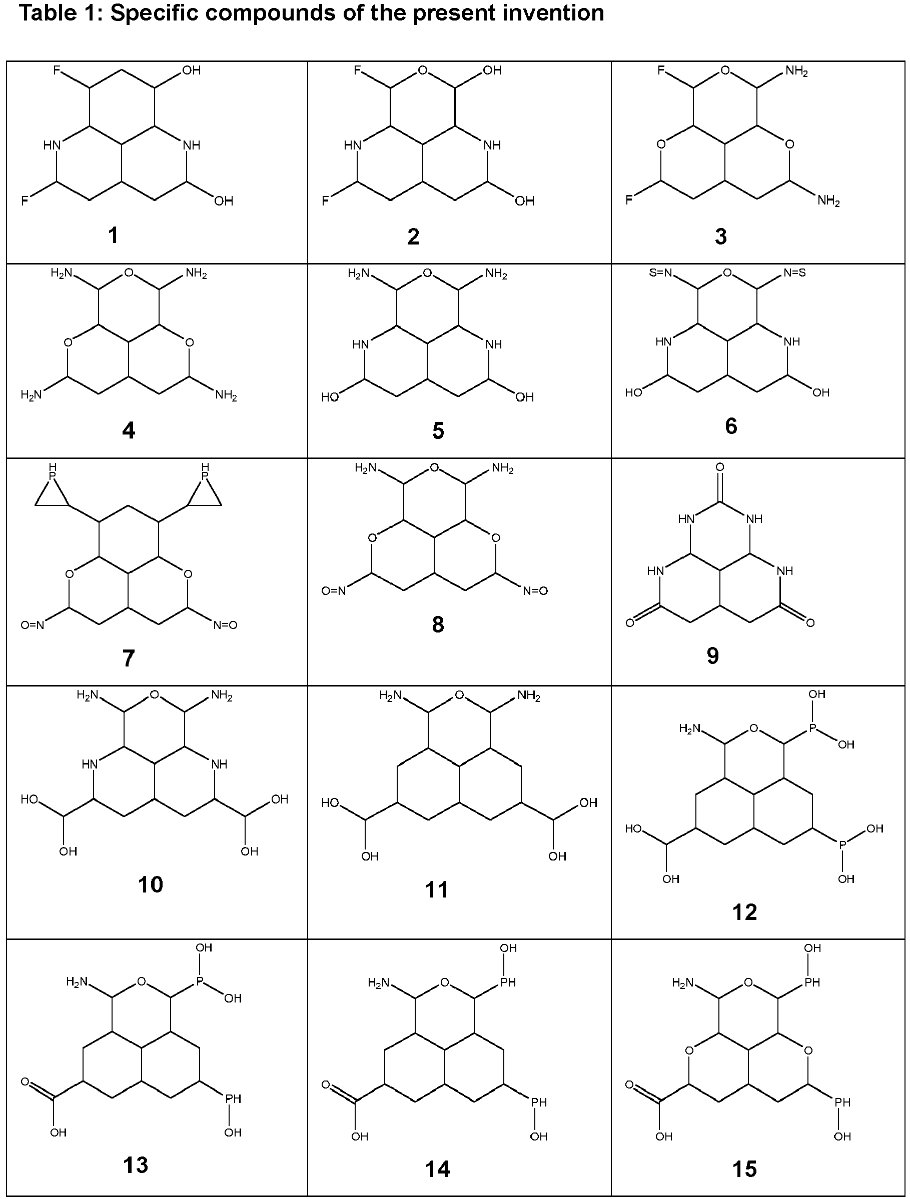

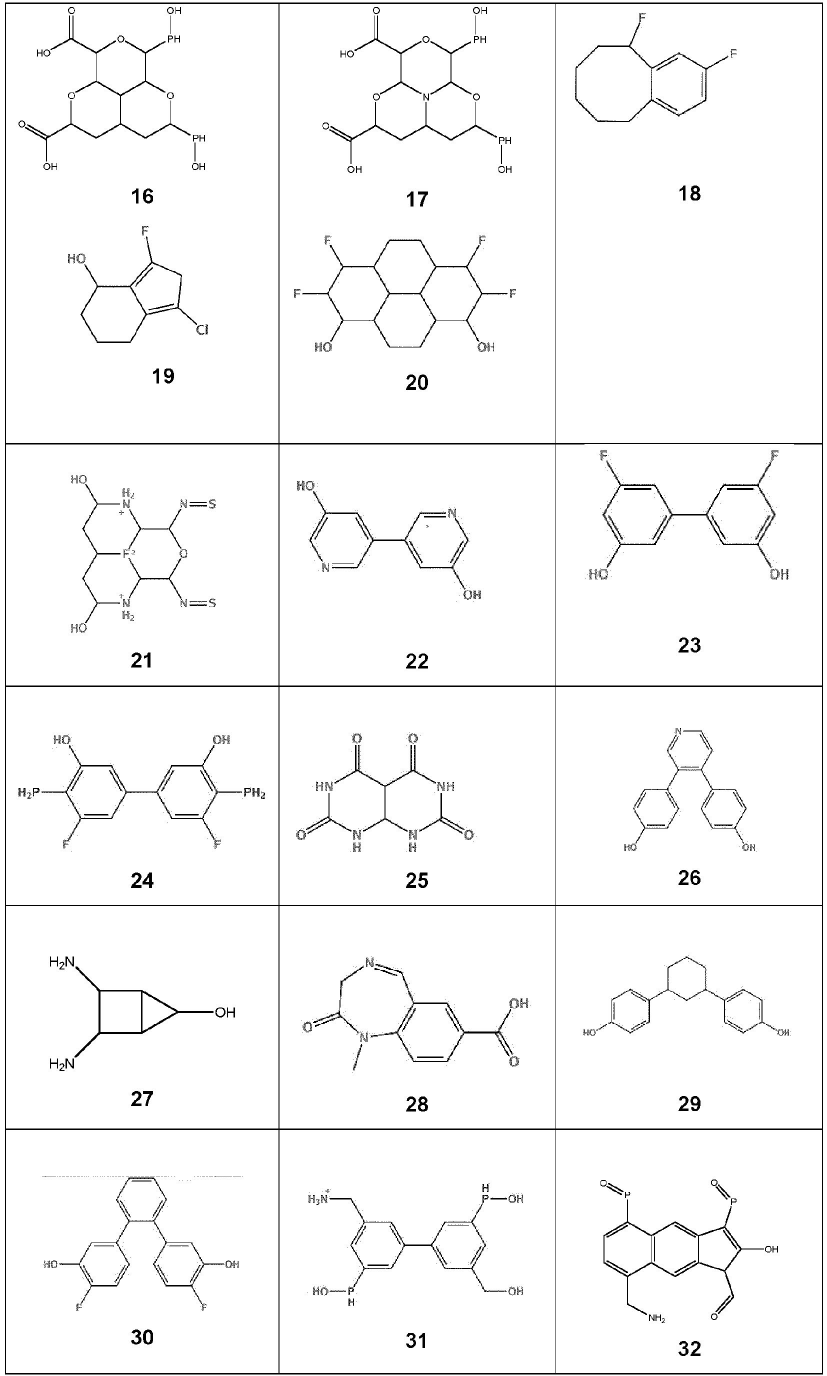

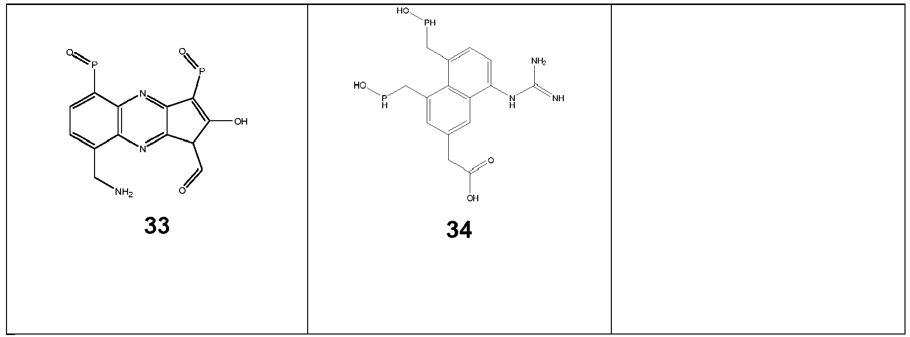

In one embodiment, the compound is selected from the group consisting of 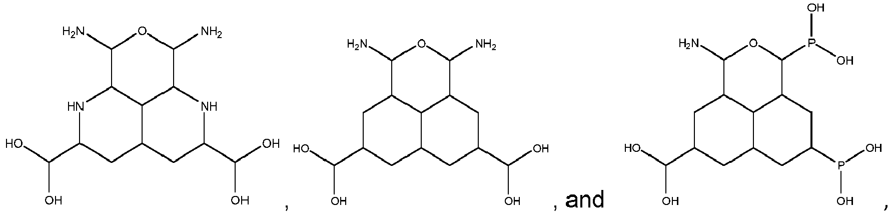 preferably 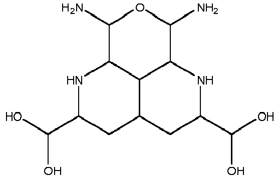 or a pharmaceutically acceptable salt, solvate or hydrate thereof.

### Pharmaceutical Compositions

In a further aspect, the present invention provides a pharmaceutical composition comprising a compound as specified above under the heading "Compounds" and one or more pharmaceutically acceptable excipients.

The compounds described in the present invention (e.g. having the general formula (I), as well as the compounds of table 1) are preferably administered to a patient in need thereof via a pharmaceutical composition. In one embodiment, the pharmaceutical composition comprises a compound as described above, as well as the compounds of table 1 or a hydrate, solvate, salt, complex, racemic mixture, diastereomer, enantiomer, or tautomer thereof (or an isotopically enriched form of any of the foregoing) and one or more pharmaceutically acceptable excipients.

The pharmaceutical composition may be administered to an individual by any route, such as enterally or parenterally.

The expressions "enteral administration" and "administered enterally" as used herein mean that the drug administered is taken up by the stomach and/or the intestine. Examples of enteral administration include oral and rectal administration. The expressions "parenteral administration" and "administered parenterally" as used herein mean modes of administration other than enteral administration, usually by injection or topical application, and include, without limitation, intravenous, intramuscular, intraarterial, intrathecal, intracapsular, intraosseous, intraorbital, intracardiac, intradermal, intraperitoneal, transtracheal, subcutaneous, subcuticular, intraarticular, subcapsular, intracerebral, intracerebroventricular, subarachnoid, intraspinal, epidural and intrasternal administration (such as by injection and/or infusion) as well as topical administration (e.g., epicutaneous, inhalational, or through mucous membranes (such as buccal, sublingual or vaginal)).

The compounds used in to the present invention (e.g. having the general formula (I), as well as the compounds of table 1) are generally applied in "pharmaceutically acceptable amounts" and in "pharmaceutically acceptable preparations". Such compositions may contain salts, buffers, preserving agents, carriers and optionally other therapeutic agents.

The term "excipient" when used herein is intended to indicate all substances in a pharmaceutical composition which are not active ingredients (e.g., which are therapeutically inactive ingredients that do not exhibit any therapeutic effect in the amount/concentration used), such as, e.g., carriers, binders, lubricants, thickeners, surface active agents, preservatives, emulsifiers, buffers, flavoring agents, colorants, or antioxidants.

The compositions described in the present invention may comprise a pharmaceutically acceptable carrier. As used herein, "pharmaceutically acceptable carrier" includes any and all solvents, dispersion media, coatings, isotonic and absorption delaying agents, and the like that are physiologically compatible. The "pharmaceutically acceptable carrier" may be in the form of a solid, semisolid, liquid, or combinations thereof. Preferably, the carrier is suitable for enteral (such as oral) or parenteral administration (such as intravenous, intramuscular, subcutaneous, spinal or epidermal administration (e.g., by injection or infusion)). Depending on the route of administration, the active compound, i.e., the compound used in the present invention, either alone or in combination with one or more additional active compounds, may be coated in a material to protect the active compound(s) from the action of acids and other natural conditions that may inactivate the active compound.

Examples of suitable aqueous and non-aqueous carriers which may be employed in the pharmaceutical compositions used according to the present invention include water (e.g., water for injection), ethanol, polyols (such as glycerol, propylene glycol, polyethylene glycol, and the like), aqueous solutions of a salt, carbohydrate, sugar alcohol, or an amino acid (such as saline or an aqueous amino acid solution), and suitable mixtures and/or buffered forms thereof, vegetable oils (such as olive oil), and injectable organic esters (such as ethyl oleate). Proper fluidity can be maintained, for example, by the use of coating materials, such as lecithin, by the maintenance of the required particle size in the case of dispersions, and by the use of surfactants.

Pharmaceutically acceptable carriers include sterile aqueous solutions or dispersions and sterile powders for the extemporaneous preparation of sterile injectable solutions or dispersions. The use of such media and agents for pharmaceutically active compounds is known in the art. Except insofar as any conventional media or agent is incompatible with the active compound, use thereof in the pharmaceutical compositions used according to the present invention is contemplated.

Additional active compounds can be administered together with, before or after the compound used in the present invention. In one embodiment, the pharmaceutical composition described herein comprises a compound as described above (e.g. having the general formula (I), as well as the compounds of table 1) or a hydrate, solvate, salt, complex, racemic mixture, diastereomer, enantiomer, or tautomer thereof or an isotopically enriched form of any of the foregoing), at least one additional active compound, and one or more pharmaceutically acceptable excipients.

The "additional active compound" (e.g. having the general formula (I), as well as the compounds of table 1) may be selected from any compound which can be used in the treatment of cancer and/or immune diseases. The additional active compound may induce an additive or synergistic therapeutic effect.

The pharmaceutical composition described herein may comprise, in addition to the compound having a structure according to formula (I), or the compounds of table 1 as described above, at least one, e.g., 1, 2, 3, 4, 5, 6, 7 or 8, additional active compounds. According to the present teaching, the at least one additional active compound, for example the anticancer drug, and/or an agent for the treatment of immune diseases, may be formulated together with the compound having a structure according to formula (I), or the compounds of table 1 as described above in a single pharmaceutical composition. Alternatively, the pharmaceutical composition may be structured as kit of parts, wherein the compound having a structure according to formula (I), or the compounds of table 1 is provided in a first formulation and the at least one additional active compound, for example the anticancer drug and/or an agent for the treatment of immune diseases, is provided in a second formulation, i.e., a second pharmaceutical composition. The first and the second pharmaceutical compositions may be combined prior to use. In other words, before administering the pharmaceutical composition, a formulation comprising the additional active compound may be added to the first pharmaceutical composition comprising the compound having a structure according to formula (I), or the compounds of table 1 as described above. Alternatively, the present teaching envisages administering the compound having a structure according to formula (I), or the compounds of table 1 as described above, formulated in a first pharmaceutical composition and administering the at least one additional active compound formulated in a second pharmaceutical composition. The pharmaceutical compositions may be administered concomitantly or in succession. For example, the first pharmaceutical composition may be administered at a first point in time and the second pharmaceutical composition may be administered at a second point in time, wherein the points in time may be separated by, for example, 0, or up to 1, 2, 3, 4, 5 or 10 min, up to 1, 2, 3, 4, 5 or 10 hours, up to 1, 2, 3, 4, 5 or 10 days, up to 1, 2, 3, 4, 5 or 10 weeks, up to 1, 2, 3, 4, 5 or 10 months or up to 1, 2, 3, 4, 5 or 10 years. In some embodiments the kit of parts comprises a compound having a structure according to formula (I), or the compounds of table 1 as described above, or a pharmaceutical composition containing a compound having a structure according to formula (I), or the compounds of table 1 as described above, and at least one pharmaceutically acceptable carrier.

The compositions may also contain adjuvants such as preservatives, wetting agents, emulsifying agents, pH buffering agents, and dispersing agents. Prevention of the presence of microorganisms may be ensured by sterilization procedures and/or by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Regardless of the route of administration selected, the active compounds, which may be used in a suitable hydrated form, and/or the pharmaceutical compositions used according to the present invention, are formulated into pharmaceutically acceptable dosage forms by conventional methods known to those of skill in the art (cf., e.g., Remington, "The Science and Practice of Pharmacy" edited by Allen, Loyd V., Jr., 22nd edition, Pharmaceutical Sciences, September 2012; Ansel et al., "Pharmaceutical Dosage Forms and Drug Delivery Systems", 7th edition, Lippincott Williams & Wilkins Publishers, 1999.).

A pharmaceutical composition can be administered by a variety of methods known in the art. As will be appreciated by the skilled artisan, the route and/or mode of administration will vary depending upon the desired results. The pharmaceutical compositions containing one or more active compounds can be prepared with carriers that will protect the one or more active compounds against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Methods for the preparation of such compositions are generally known to those skilled in the art. See, e.g., Sustained and Controlled Release Drug Delivery Systems, J. R. Robinson, ed., Marcel Dekker, Inc., New York, 1978.

To administer a compound used in the present invention by certain routes of administration, it may be necessary to coat the compound with, or co-administer the compound with, a material to prevent its inactivation. For example, the compound may be administered to an individual in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al., J. Neuroimmunol. 7: 27(1984)).

Pharmaceutical compositions typically are sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

An injectable composition should be sterile and fluid to the extent that the composition is deliverable by syringe. In addition to water, the carrier can be an isotonic buffered saline solution, ethanol, polyol (for example, glycerol, propylene glycol, and liquid polyethylene glycol, and the like), and suitable mixtures thereof. Sterile injectable solutions can be prepared by incorporating the active compound in the required amount in an appropriate solvent with one or a combination of ingredients enumerated above, as required, followed by sterilization microfiltration.

Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the individuals to be treated; each unit contains a predetermined quantity of active compound calculated to produce the desired therapeutic effect in association with the required pharmaceutical carrier. The specification for the dosage unit forms used according to the present invention are dictated by and directly dependent on (a) the unique characteristics of the active compound and the particular therapeutic effect to be achieved, and (b) the limitations inherent in the art of compounding such an active compound for the treatment of sensitivity in individuals.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

For the therapeutic/pharmaceutical formulations, compositions used according to the present invention include those suitable for enteral administration (such as oral or rectal) or parenteral administration (such as nasal, topical (including vaginal, buccal and sublingual)). The compositions may conveniently be presented in unit dosage form and may be prepared by any methods known in the art of pharmacy. The amount of active ingredient (in particular, the amount of a compound used according to the present invention) which can be combined with a carrier material to produce a pharmaceutical composition (such as a single dosage form) will vary depending upon the individual being treated, and the particular mode of administration. The amount of active ingredient which can be combined with a carrier material to produce a single dosage form will generally be that amount of the composition which produces a therapeutic effect.

Generally, out of 100% (for the pharmaceutical formulations/compositions), the amount of active ingredient (in particular, the amount of the compound used according to the present invention, optionally together with other therapeutically active agents, if present in the pharmaceutical formulations/compositions) will range from about 0.01% to about 99%, preferably from about 0.1% to about 70%, most preferably from about 1% to about 30%, wherein the reminder is preferably composed of the one or more pharmaceutically acceptable excipients.

The amount of active ingredient, e.g., a compound used according to the present invention, in a unit dosage form and/or when administered to an individual or used in therapy, may range from about 0.1 mg to about 1000 mg (for example, from about 1 mg to about 500 mg, such as from about 10 mg to about 200 mg) per unit, administration or therapy. In certain embodiments, a suitable amount of such active ingredient may be calculated using the mass or body surface area of the individual, including amounts of between about 1 mg/kg and 10 mg/kg (such as between about 2 mg/kg and 5 mg/kg), or between about 1 mg/m² and about 400 mg/m² (such as between about 3 mg/m² and about 350 mg/m² or between about 10 mg/m² and about 200 mg/m²).

Actual dosage levels of the active ingredients in the pharmaceutical compositions used according to the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start with doses of the compounds used according to the present invention at levels lower than that required in order to achieve the desired therapeutic effect and gradually increase the dosage until the desired effect is achieved. In general, a suitable daily dose of a composition used according to the present invention will be that amount of the compound which is the lowest dose effective to produce a therapeutic effect. Such an effective dose will generally depend upon the factors described above. It is preferred that administration be oral, intravenous, intramuscular, intraperitoneal, or subcutaneous, preferably administered proximal to the site of the target. If desired, the effective daily dose of a pharmaceutical composition may be administered as two, three, four, five, six or more sub-doses administered separately at appropriate intervals throughout the day, optionally, in unit dosage forms. While it is possible for a compound used according to the present invention to be administered alone, it is preferable to administer the compound as a pharmaceutical formulation/composition.

For oral administration, the pharmaceutical composition used according to the present invention can take the form of, for example, tablets or capsules prepared by conventional means with pharmaceutical acceptable excipients such as binding agents (e.g., pregelatinised maize starch, polyvinylpyrrolidone, hydroxypropyl methylcellulose), fillers (e.g., lactose, microcrystalline cellulose, calcium hydrogen phosphate), lubricants (e.g., magnesium stearate, talc, silica), disintegrants (e.g., potato starch, sodium starch glycolate), or wetting agents (e.g., sodium lauryl sulphate). Liquid preparations for oral administration can be in the form of, for example, solutions, syrups, or suspensions, or can be presented as a dry product for constitution with water or other suitable vehicle before use. Such liquid preparation can be prepared by conventional means with pharmaceutically acceptable additives such as suspending agents (e.g., sorbitol, syrup, cellulose derivatives, hydrogenated edible fats), emulsifying agents (e.g., lecithin, acacia), non-aqueous vehicles (e.g., almond oil, oily esters, ethyl alcohol, fractionated vegetable oils), preservatives (e.g., methyl or propyl-p-hydroxycarbonates, sorbic acids). The preparations can also contain buffer salts, flavouring, coloring and sweetening agents as deemed appropriate. Preparations for oral administration can be suitably formulated to give controlled release of the pharmaceutical composition of the invention.

In one embodiment, the compound is orally administered in a concentration of at most 100 mg/kg body weight (such as at most 50 mg/kg body weight, at most 40 mg/kg body weight, at most 30 mg/kg body weight, at most 20 mg/kg body weight, at most 10 mg/kg body weight, at most 5 mg/kg body weight, at most 4 mg/kg body weight, at most 3 mg/kg body weight, at most 2 mg/kg body weight, at most 1 mg/kg body weight).

In one embodiment, the compound is parenterally administered (e.g., intravenously, intramuscularly, or subcutaneously), in a concentration of at most 10 mg/kg body weight (such as at most 5 mg/kg body weight, at most 4 mg/kg body weight, at most 3 mg/kg body weight, at most 2 mg/kg body weight, at most 1 mg/kg body weight, at most 0.5 mg/kg body weight, at most 0.4 mg/kg body weight, at most 0.3 mg/kg body weight, at most 0.2 mg/kg body weight, at most 0.1 mg/kg body weight).

The pharmaceutical composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc.

The pharmaceutical composition used according to the present invention can be formulated for parenteral administration by injection, for example, by bolus injection or continuous infusion. In one embodiment, the compounds or compositions used according to the present invention may be administered by slow continuous infusion over a long period, such as more than 24 hours, in order to reduce toxic side effects. The administration may also be performed by continuous infusion over a period of from 2 to 24 hours, such as of from 2 to 12 hours. Such regimen may be repeated one or more times as necessary, for example, after 6 months or 12 months.

In yet another embodiment, the compounds or compositions used according to the present invention are administered by maintenance therapy, such as, e.g., once a week for a period of 6 months or more.

Formulations for injection can be presented in units dosage form (e.g., in phial, in multidose container), and with an added preservative. The pharmaceutical composition used according to the present invention can take such forms as suspensions, solutions or emulsions in oily or aqueous vehicles, and can contain formulatory agents such as suspending, stabilizing, or dispersing agents. Alternatively, the agent can be in powder form for constitution with a suitable vehicle (e.g., sterile pyrogen-free water) before use. Typically, compositions for intravenous administration are solutions in sterile isotonic aqueous buffer. Where necessary, the composition can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

Compositions used according to the present invention which are suitable for vaginal administration also include pessaries, tampons, creams, gels, pastes, foams or spray formulations containing such carriers as are known in the art to be appropriate. Dosage forms for the topical or transdermal administration of compositions used according to the present invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

In one embodiment, the compounds used according to the present invention are formulated in liposomes. In a more preferred embodiment, the liposomes include a targeting moiety. In a most preferred embodiment, the compounds in the liposomes are delivered by bolus injection to a site proximal to the desired area. Such liposome-based composition should be fluid to the extent that easy syringability exists, should be stable under the conditions of manufacture and storage and should be preserved against the contaminating action of microorganisms such as bacteria and fungi.

A "therapeutically effective dosage" for therapy/treatment can be measured by objective responses which can either be complete or partial. A complete response (CR) is defined as no clinical, radiological or other evidence of a condition, disorder or disease. A partial response (PR) results from a reduction in disease of greater than 50%. Median time to progression is a measure that characterizes the durability of the objective tumor response.

A "therapeutically effective dosage" for therapy/treatment can also be measured by its ability to stabilize the progression of a condition, disorder or disease. The ability of a compound to inhibit TRAF6 can be evaluated by using any of the *in vitro* or cell-based assays described herein measuring the TRAF6 activity/inhibition. Alternatively, the properties of a compound described in the present invention can be evaluated by examining the ability of the compound in appropriate animal model systems known to the skilled practitioner. A therapeutically effective amount of a compound used according to the present invention can cure, heal, alleviate, relieve, alter, remedy, ameliorate, improve or affect the condition, disorder or disease or the symptoms of the condition, disorder or disease or the predisposition toward the condition, disorder or disease in an individual. One of ordinary skill in the art would be able to determine such amounts based on such factors as the individual's size, the severity of the individual's symptoms, and the particular composition or route of administration selected.

The pharmaceutical composition used according to the invention can also, if desired, be presented in a pack, or dispenser device which can contain one or more unit dosage forms containing the active compound. The pack can for example comprise metal or plastic foil, such as blister pack. The pack or dispenser device can be accompanied with instruction for administration.

The pharmaceutical composition used according to the invention can be administered as sole active agent or can be administered in combination with other therapeutically and/or cosmetically active agents. In one embodiment, the pharmaceutical composition used according to the invention contains, or is administered with, one or more other therapeutically active agents selected from the group consisting of antiviral agents, antibodies (which are directed against an antigen of an animal pathogenic virus or another microorganism (e.g., a pathogenic bacterium or fungi) or against a cancer antigen), agents stimulating the immune system of the subject (e.g., interferons, such as interferon alpha or interferon beta, imiquimod, and resiquimod), and antimicrobial agents.

### Therapeutic applications

The compounds according to present invention are for use in medicine.

In one embodiment, the compound or the pharmaceutical composition as described above is used in the prevention of the aggregation of phosphorylated Tau proteins. This would stop the pathological process altogether. The molecular dynamics simulations strongly demonstrate the effects of the compounds delineated here to prevent already and extremely early the aggregation of phosphorylated Tau proteins.

In a further embodiment, the compound or the pharmaceutical composition as described above is for use in the treatment or prevention of a tauopathy. Tauopathy is a group of neurodegenerative diseases, characterized by the presence of aggregates of phosphorylated tau proteins. For this condition, the compounds identified are able to resolve such aggregates, which means they can specifically revert the pathological process back to normal condition.

In another embodiment, the compound or the pharmaceutical composition as described above is for use in the treatment or prevention of a neurodegenerative disease, preferably selected from the group consisting of Primary age-related tauopathy (PART) dementia, Chronic traumatic encephalopathy, Progressive supranuclear palsy, Corticobasal degeneration and Alzheimer's disease, more preferably Alzheimer's disease. Here the clinical case is referred to: Patients with symptoms of a neurodegenerative disease, preferably selected from the group consisting of Primary age-related tauopathy (PART) dementia, Chronic traumatic encephalopathy, Progressive supranuclear palsy, Corticobasal degeneration and Alzheimer's disease, preferably Alzheimer's disease. The examples show that also with high concentration of aggregates the inventive compounds are effective, a therapeutic treatment option exists even for symptomatic cases of the neurodegenerative disease, preferably selected from the group consisting of Primary age-related tauopathy (PART) dementia, Chronic traumatic encephalopathy, Progressive supranuclear palsy, Corticobasal degeneration and Alzheimer's disease, preferably Alzheimer's disease. Moreover, even with different concentrations of tau protein aggregates the further formation of these aggregates is stopped, thus further progression of the neurodegenerative disease, preferably selected from the group consisting of Primary age-related tauopathy (PART) dementia, Chronic traumatic encephalopathy, Progressive supranuclear palsy, Corticobasal degeneration and Alzheimer's disease, preferably Alzheimer's disease is prevented and treated by reversion of the underlying pathological condition such as a tauopathy as described already above.

### EXAMPLES OF THE INVENTION

### Methods

### Structure derivation and preparation.

The crystal structure of MT in a complex with Tau peptide was retrieved from protein data bank (PDB) (http://www.rcsb.org) under the 6CVJ reference code¹. The protein structure preparation as well as phosphorylation of serine residues of Tau at positions 258 and 262 was performed with the help of MOE software¹¹.

### Design of the compounds.

All compounds were designed *de novo* taking into account the shape of the cavity formed between the phosphoserine of tau peptide and the corresponding residues of microtubule. Design of the compounds was performed with the help of MOE software¹¹.

### Docking.

Molecular docking simulations have been performed with the help of well-validated^{,13} software such as GOLD¹⁴, MOE¹¹ and AutoDock¹².

**GOLD.** Molecular docking using GOLD software was performed using version 5.5¹⁴. Binding site residues were defined by specifying the crystal structure ligand coordinates bound to the protein and using the default cutoff radius of 6 Å, with the "detect cavity" option enabled. GOLD docking experiments were performed using the ChemPLP scoring function. For each compound, 50 complexes were generated. The highest scoring compounds were selected as the most appropriate ones.

**MOE.** Docking has been performed by selecting the default "Rigid Receptor" protocol. As a binding site, the coordinates of co-crystalized ligand atoms have been selected. The ligand placement was performed using the Triangle Matcher protocol. The top 30 poses were ranked by London dG scoring function and the resulting 5 poses were identified using Generalized-Born Volume Integral / Weighted Surface Area (GBVI/WSA dG) function. The conformations with more negative final score were considered as favorables.

**AutoDock.** Molecular docking using AutoDock software¹² has been performed using version 4.2.6, available at http://autodock.scripps.edu. The AutoDock Tools (ADT) were used to generate input parameter files for docking. For the current study the receptor was considered as a rigid molecule, while ligands contained rotatable bonds. Only protein was considered for the docking, while all non-protein moieties were removed. Additional hydrogen atoms were added to the receptor and the new PDB coordinates saved. The ligand PDB file was modified by addition of groups representing the Gasteiger charges. The volume of the grid box was set as 50 x 50 x 50 Å with 0.375 Å spacing. The center of the grid box was placed so that it coincided with the center of the phosphorylated serine residue of tau protein. A genetic algorithm was selected to set the search parameters. The number of docking runs was fixed to 50. The conformations with the lowest binding energies have been selected for further analysis.

The figures were prepared using UCSF Chimera¹⁵ (available at http://www.rbvi.ucsf.edu/chimera) and MOE¹¹ software.

### Molecular dynamics simulations.

**System Setup.** The 6 Tau peptides were centered in cubic boxes of size 7 nm and the empty space was filled with water molecules. The water molecules within 0.25 nm from the solute atoms were removed. Na⁺/Cl⁻ counter ions were added to keep the system neutral. To understand the effect of phosphorylation on Tau aggregation, the first two sets of MD simulations were performed on pure and phosphorylated at Ser 262 and Ser 258 Tau peptides in water. The next two sets of simulations were performed for Tau peptides with the presence of the designed compound 10. And, finally, two last sets were performed for the phosphorylated Tau bundles in the presence of compound 10. The Tau bundles were extracted from the previous simulation of Tau peptides in water. For water, the simple point charge (SPC) model was used in all the simulations. For the Tau peptide and ions the GROMOS54a7¹⁶⁴⁴ force field was used. The extended force field for the phosphorylated Tau peptides was generated with the help of Vienna-PTM 2.0 online tool^{,17}.

**MD run.** MD simulations were performed using GROMACS¹⁷ (version 2019.3) software package. The LINCS algorithm¹⁸ was applied in order to constraint the bond lengths during the simulation. The integration time step was set to 2 fs. The temperature and pressure were maintained to the reference values (T=300 and 310 K, P=1 bar) using the V-rescale thermostat¹⁹ and Parrinello - Rahman barostat²⁰ with the coupling time constant of τT = 0.1 ps for temperature and τp = 2 ps for pressure, respectively. The isothermal compressibility of 4.5 × 10⁻⁵ bar⁻¹ was used for all the simulation. Particle mesh Ewald (PME) method²¹ was applied for the long-range interactions with a real space cutoff of 1 nm, a Fourier mesh spacing of 0.16 nm. The Lennard-Jones interactions were calculated using the cutoff of 1.4 nm. The protonation state of the peptide amino acids in all simulations was assumed the one at neutral pH. All the systems were initially energy minimized using steepest descent algorithm of at least 1000 steps in order to relax the system and remove steric clashes among atoms. Prior to submission for the final MD run, the systems where first equilibrated using NVT and NPT ensembles for 100 ps. The length of the final production run as well as the summary of composition of each simulation is reported in **Table 2**.

**Table 2. Composition and temperature conditions of the simulated systems.***

| | **System Reference Code** | **No. of Tau peptides** | **No. of Compounds** | **No. of Water Molecules** | **No. of CL⁻ ions** | **No. of NA⁺ ions** | **Temp., K** | **Simulation length, ns** |
|---|---|---|---|---|---|---|---|---|
| 1 | T | 6 | 0 | 10875 | 0 | 0 | 300 | 250 |
| 2 | T_{P} | 6 | 0 | 10862 | 0 | 0 | 300 | 250 |
| 3 | T_{P}C₂₃300 | 6 | 30 | 10322 | 120 | 0 | 300 | 250 |
| 4 | T_{P}C₂₃310 | 6 | 30 | 10322 | 120 | 0 | 310 | 250 |
| 5 | T_{P}C₃₁300 | 6 | 30 | 10409 | 0 | 30 | 310 | 250 |
| 6 | T_{P}C₃₁310 | 6 | 30 | 10409 | 0 | 30 | 310 | 250 |
| 7 | T_{P}BC₂₃310 | 6 | 30 | 10335 | 120 | 0 | 310 | 500 |
| 8 | T_{P}BC₃₁310 | 6 | 30 | 10424 | 0 | 30 | 310 | 500 |
| 9 | T_{P}BC₂₃310 | 6 | 60 | 9792 | 240 | 0 | 310 | 500 |
| 10 | T_{P}BC₃₁310 | 6 | 60 | 9987 | 0 | 60 | 310 | 500 |

*The numbers and lower case "p" letter on the right side of the capital letters indicate the phosphorylated Tau peptides, the compound consecutive number and the temperature in the simulation, respectively. The capital letter stands for: T - Tau peptide; Tₚ - phosphorylated Tau peptide; C₂₃ - compound 10, B - bundle composed of phosphorylated Tau peptides, respectively. Example: T_{P}C₂₃300 is the simulation of a mixture composed of phosphorylated Tau peptides and compound 10 in water at 300 K. T_{P}BC₂₃310 is the simulation of a phosphorylated Tau bundle in water at 310 K in the presence of compound 10.

### Results

The interaction of Tau with MT is significant to maintain the stability of the latter one and avoid the development of neurodegenerative diseases^{2,3}. Genetic mutations as well as abnormal phosphorylation may impair the Tau-MT interaction leading towards Tau detachment and destabilization of MTs²,⁷ from one side, while from the other side, this can affect the process of Tau-MT assembly^{3, 5,6,}. Therefore, here both possibilities have been considered and docking of the designed compounds has been performed taking into account Tau-MT complex as well as single Tau peptide. For better estimation and higher accuracy, the results performed by three independent docking algorithms (AutoDock, MOE and GOLD) have been provided. **Table 3** reports the scores for the *de novo* designed compounds. In the first colon of the table the number of the compound is provided, in the second one the 2D structure of the compound, while the third, fourth and fifth colons show the docking scores according to three docking protocols - AutoDock, MOE and GOLD. The last three colons, in turn, are also divided into sub colons and report the docking scores for the compounds with respect to Tau-associated MT complex as well as single Tau peptide (colons highlighted in green). As results show, the compounds 10, 11 and 12 exhibit the highest scores against both Tau-MT complex and single Tau peptide (see **Table 3**. Compound 10 shows the highest score amongst all studied compounds according to AutoDock software. Compounds 11 and 12 are similar to compound 10, however, they do not show significant difference in scores compared to compound 10. Therefore, for further tests compound 10 was selected, and molecular dynamics simulation studies were performed to understand their influence on the phenomenon of Tau aggregation as well as their strength in tau de-bundling.

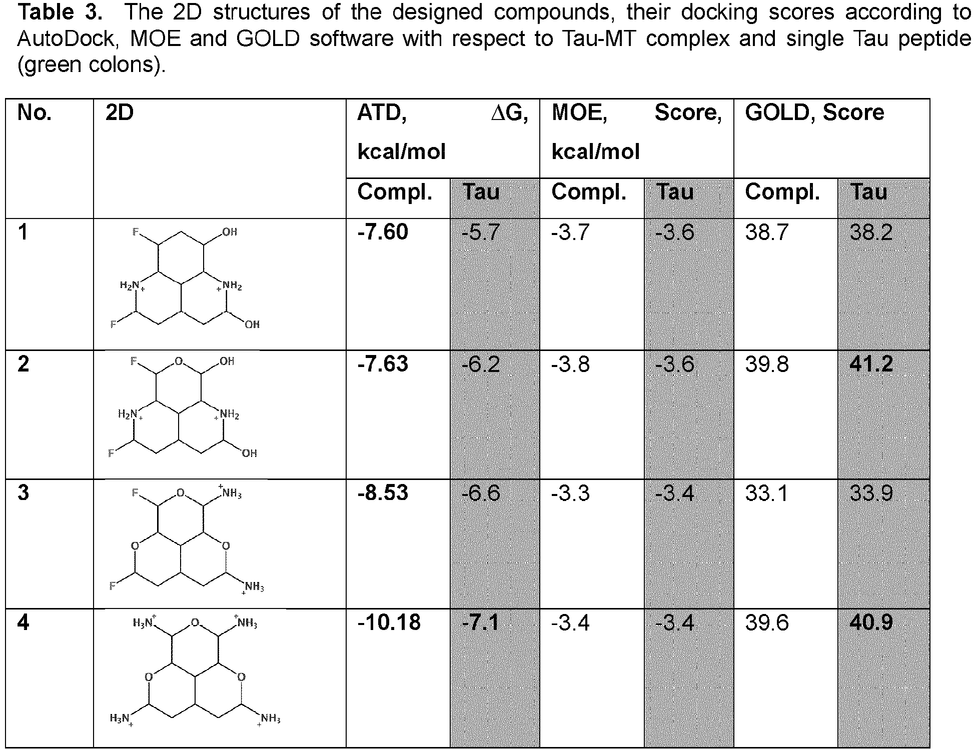

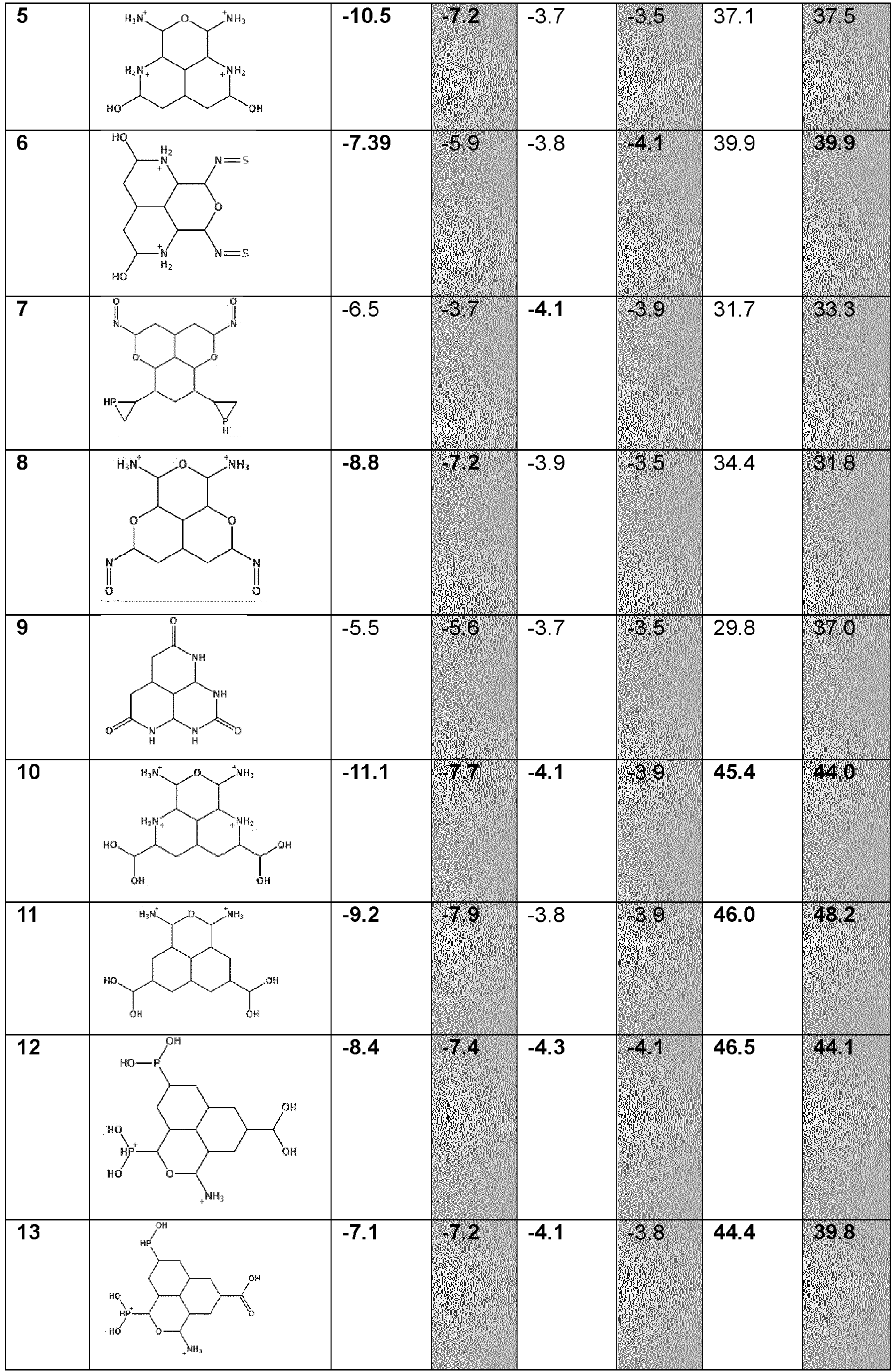

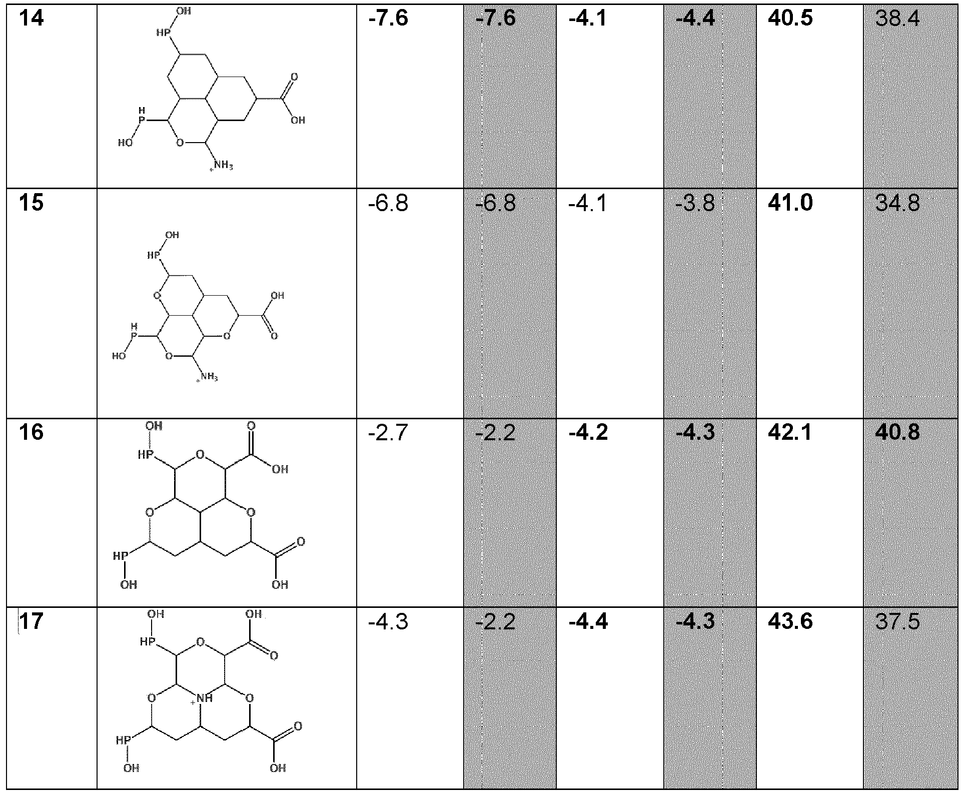

**Figure 1** demonstrates the complexes of compound 10 (A, B) associated with Tau-MT and single Tau peptide, respectively. As the figure shows, the compound forms H-bond interactions with the key phosphorylated serines (S262, S258).

### Molecular dynamics simulations

### a. Effect of Tau phosphorylation on its aggregation

To evaluate the influence of phosphorylation on tau aggregation two systems have been compared: one with the native Tau peptides in a box of water, another - with a Tau phosphorylated at serine residues (Ser262 and Ser258). **Figure 2** shows snapshots for the native Tau peptides taken at 0 ns, 5 ns, 10 ns, 50 ns, 150 and 250 ns simulation time, while **Figure 3** represents the 3D bars for the minimum distances vs. time frames between the backbones of peptide pairs. The results show that the Tau peptides start aggregation from 5 ns. However, the phenomenon of aggregation is not consistent in time. In particular, the peptides associate into aggregates and disassociate again. Even by 250 ns there is no stable formation of a bundle. In contrast, the system, where the phosphorylated Tau peptides have been placed has been compared. In this case the formation of a bundle starts from 5 ns and remains stable from 50 ns till the end of the simulation time - 250 ns. This is particularly obvious from the **Figures 4** and **5** corresponding to the dynamics of phosphorylated Tau in time and minimum distances between the backbones of peptide pairs, respectively. Compared to **Figure 3****,** **Figure 5** shows the significant decrease in distances between the peptide pairs at different time frames, which indicates on their aggregation. Such a behavior of Tau peptides confirms the phenomenon observed experimentally, namely, that phosphorylation promotes the aggregation of Tau into bundles²².

### b. Influence of the designed compounds on Tau aggregation

Next, the influence of the high-scoring compound 10 on the prevention of phosphorylated Tau from aggregation has been studied. As in the previous case two simulation boxes have been constructed with phosphorylated Tau peptides and compound 10 and the boxes were filled up with water. After completion of simulation the dynamics of Tau in the two boxes were compared. It has been found that the presence of compound 10 significantly reduces the aggregation of Tau peptides into a bundle (see **Figures 6** and **7**). The peptides do not form stable aggregates. Instead, they associate and disassociate during the simulation time. Concerning the behavior of compound 10 itself, it tries to coat the surface of the peptides, hence, reducing their contact with each other and preventing them from aggregation. The compounds themselves remain in solution in an isolated state (see **Figure 6**). The peptides aggregate stronger than in the case of influence of compound 10. The temperature increase until 310 K (37 °C) did not show any drastic changes in peptide behavior in both cases. Instead, this only accelerated the dynamics (see Figure 12).

### c. Influence of the designed compounds on tau de-bundling: low concentration of compounds

Finally, the strength of the above-mentioned compounds on de-bundling of the Tau peptides has been tested. For this, the bundle, which has formed during the simulation of phosphorylated Tau peptides in water has been extracted, and placed in two separate simulation boxes: one with the presence of compound 10 and the control without compound 10. Both boxes were filled up with water and subjected to the simulation run of 500 ns. **Figure 8** shows the dynamic of the bundle in time with the presence of compound 10 at 0, 50, 100, 150, 250, 350, 450 and 500 ns. As the figure shows, starting from 50 ns the compounds try to separate the peptides from each other. Such a phenomenon can also be observed at 250 and 350 ns time frame (**Figures 8** and **9** (blue and green bars)). However, this process is not constant in time: the peptides disassemble and reassemble again, which indicates on the necessity of stronger force for bundle separation.

### d. Influence of the designed compounds on tau de-bundling: high concentration of compounds

Next, to increase the acting force on the bundle it has been decided to double the concentration of the compounds. **Figures 10** shows the behavior of the bundle at different time frames under the influence of compound 10. As expected, with the increase of the concentration the acting force on the bundle has increased as well, which in turn, facilitated the de-bundling process. It can be easily observed that starting from 50 ns one peptide separates from the bundle; at 100 ns there are three separate peptide pairs; further separation takes place at 450 ns and peptides/pairs remain separate till the end of the simulation time. **Figure 11** shows the 3D bars for the minimum distance between the backbones of peptide pairs in a bundle taken at corresponding time frames. The increase of distance between the pairs, that is obvious from the **Figure 11**, also indicates the effectiveness of compound 10 on peptide separation.

### REFERENCES

(1) Kellogg, E. H.; Hejab, N. M. A.; Poepsel, S.; Downing, K. H.; DiMaio, F.; Nogales, E. Near-Atomic Model of Microtubule-Tau Interactions. Science (80-. ). 2018, 360 (6394), 1242-1246. https://doi.org/10.1126/science.aat1780.
(2) Kadavath, H.; Hofele, R. V.; Biernat, J.; Kumar, S.; Tepper, K.; Urlaub, H.; Mandelkow, E.; Zweckstetter, M. Tau Stabilizes Microtubules by Binding at the Interface between Tubulin Heterodimers. Proc. Natl. Acad. Sci. U. S. A. 2015, 112 (24), 7501-7506. https://doi.org/10.1073/pnas.1504081112.
(3) Sergeant, N.; Delacourte, A.; Buée, L. Tau Protein as a Differential Biomarker of Tauopathies. Biochimica et Biophysica Acta - Molecular Basis of Disease. 2005, pp 179-197. https://doi.org/10.1016/j.bbadis.2004.06.020.
(4) Barbier, P.; Zejneli, O.; Martinho, M.; Lasorsa, A.; Belle, V.; Smet-Nocca, C.; Tsvetkov, P. O.; Devred, F.; Landrieu, I. Role of Tau as a Microtubule-Associated Protein: Structural and Functional Aspects. Frontiers in Aging Neuroscience. 2019. https://doi.org/10.3389/fnagi.2019.00204.
(5) Biernat, J.; Gustke, N.; Drewes, G.; Mandelkow, E.; Mandelkow, E. Phosphorylation of Ser262 Strongly Reduces Binding of Tau to Microtubules: Distinction between PHF-like Immunoreactivity and Microtubule Binding. Neuron 1993, 11 (1), 153-163. https://doi.org/10.1016/0896-6273(93)90279-Z.
(6) Bramblett, G. T.; Goedert, M.; Jakes, R.; Merrick, S. E.; Trojanowski, J. Q.; Lee, V. M. Y. Abnormal Tau Phosphorylation at Ser396 in Alzheimer's Disease Recapitulates Development and Contributes to Reduced Microtubule Binding. Neuron 1993, 10 (6), 1089-1099. https://doi.org/10.1016/0896-6273(93)90057-X.
(7) Lee Virginia, M. Y; Zhukareva, V.; Vogelsberg-Ragaglia, V.; Wszolek, Z.; Reed, L.; Miller, B. I.; Geschwind, D. H.; Bird, T. D.; McKeel, D.; Coate, A.; Morris, J. C.; Wilhelmsen, K. C.; Schellenberg, G. D.; Trojanowski, J. Q.; Lee, V. M. Y. Mutation-Specific Functional Impairments in Distinct Tau Isoforms of Hereditary FTDP-17. Science (80-. ). 1998, 282 (5395), 1914-1917. https://doi.org/10.1126/science.282.5395.1914.
(8) Del C. Alonso, A.; Grundke-lqbal, I.; Iqbal, K. Alzheimer's Disease Hyperphosphorylated Tau Sequesters Normal Tau into Tangles of Filaments and Disassembles Microtubules. Nat. Med. 1996, 2 (7), 783-787. https://doi.org/10.1038/nm0796-783.
(9) Altmann, K. H. Microtubule-Stabilizing Agents: A Growing Class of Important Anticancer Drugs. Current Opinion in Chemical Biology. 2001, pp 424-431. https://doi.org/10.1016/S1367-5931(00)00225-8.
(10) Hung, S. Y; Fu, W. M. Drug Candidates in Clinical Trials for Alzheimer's Disease. Journal of Biomedical Science. 2017. https://doi.org/10.1186/s12929-017-0355-7.
(11) Molecular Operating Environment (MOE), 2013.08. Molecular Operating Environment (MOE), 2013.08; Chemical Computing Group Inc., 1010 Sherbooke St. West, Suite #910, Montreal, QC, Canada, H3A 2R7. Mol. Oper. Environ. (MOE), 2013.08; Chem. Comput. Gr. Inc., 1010 Sherbooke St. West, Suite #910, Montr. QC, Canada, H3A 2R7, 2013. 2016.
(12) Forli, S.; Huey, R.; Pique, M. E.; Sanner, M. F.; Goodsell, D. S.; Olson, A. J. Computational Protein-Ligand Docking and Virtual Drug Screening with the AutoDock Suite. Nat. Protoc. 2016, 11 (5), 905-919. https://doi.org/10.1038/nprot.2016.051.
(13) Chan, S. L.; Labute, P. Training a Scoring Function for the Alignment of Small Molecules. J. Chem. Inf. Model. 2010, 50 (9), 1724-1735. https://doi.org/10.1021/ci100227h.
(14) Jones, G.; Willett, P.; Glen, R. C.; Leach, A. R.; Taylor, R. Development and Validation of a Genetic Algorithm for Flexible Docking. J. Mol. Biol. 1997, 267 (3), 727-748. https://doi.org/10.1006/jmbi.1996.0897.
(15) Pettersen, E. F.; Goddard, T. D.; Huang, C. C.; Couch, G. S.; Greenblatt, D. M.; Meng, E. C.; Ferrin, T. E. UCSF Chimera-A Visualization System for Exploratory Research and Analysis. J Comput Chem 2004, 25, 1605-1612. https://doi.org/10.1002/jcc.20084.
(16) Schmid, N.; Eichenberger, A. P.; Choutko, A.; Riniker, S.; Winger, M.; Mark, A. E.; Van Gunsteren, W. F. Definition and Testing of the GROMOS Force-Field Versions 54A7 and 54B7. Eur. Biophys. J. 2011, 40 (7), 843-856. https://doi.org/10.1007/s00249-011-0700-9.
(17) Margreitter, C.; Reif, M. M.; Oostenbrink, C. Update on Phosphate and Charged Post-Translationally Modified Amino Acid Parameters in the GROMOS Force Field. J. Comput. Chem. 2017, 38 (10), 714-720. https://doi.org/10.1002/jcc.24733.
(18) Hess, B.; Bekker, H.; Berendsen, H. J. C.; Fraaije, J. G. E. M. LINCS: A Linear Constraint Solver for Molecular Simulations. J. Comput. Chem. 1997, 18 (12), 1463-1472. https://doi.org/10.1002/(SICI)1096-987X(199709)18:12<1463::AID-JCC4>3.0.CO;2-H.
(19) Bussi, G.; Donadio, D.; Parrinello, M. Canonical Sampling through Velocity Rescaling. J. Chem. Phys. 2007, 126 (1). https://doi.org/10.1063/1.2408420.
(20) Parrinello, M.; Rahman, A. Polymorphic Transitions in Single Crystals: A New Molecular Dynamics Method. J. Appl. Phys. 1981, 52 (12), 7182-7190. https://doi.org/10.1063/1.328693.
(21) Darden, T.; York, D.; Pedersen, L. Particle Mesh Ewald: An N·log(N) Method for Ewald Sums in Large Systems. J. Chem. Phys. 1993, 98 (12), 10089-10092. https://doi.org/10.1063/1.464397.
(22) Alonso, A. D.; Cohen, L. S.; Corbo, C.; Morozova, V.; Elldrissi, A.; Phillips, G.; Kleiman, F. E. Hyperphosphorylation of Tau Associates with Changes in Its Function beyond Microtubule Stability. Frontiers in Cellular Neuroscience. 2018. https://doi.org/10.3389/fncel.2018.00338.

## Claims

1. A compound according to formula (I) 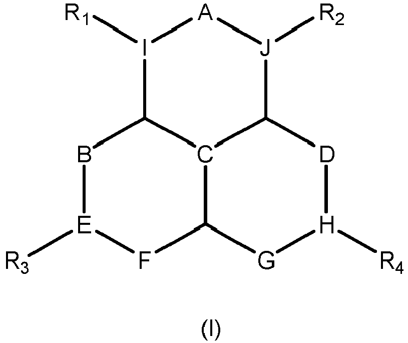 wherein
R₁ is selected from the group consisting of a) absent if I is CH₂ or
b) halogen, -OH, -NH₂, -N=S, -P(OH)₂, -PH(OH), 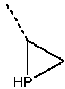 and -COOH if I is CH;
R₂ is selected from the group consisting of a) absent if J is CH₂ or
b) halogen, -OH, -NH₂, -N=S, -P(OH)₂, -PH(OH), 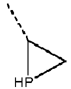 and -COOH if J is CH;
R₃ is selected from the group consisting of a) absent if E is CH₂; or
b) halogen, and -COOH , -PH(OH), -P(OH)₂, -OH, - NH₂, -CH(OH)₂, -N=O if E is CH; or
c) =O if E is C;
R₄ is selected from the group consisting of a) absent if H is CH₂; or
b) halogen, -COOH, -CH(OH)₂, -PH(OH), -P(OH)₂, - CH(OH)₂, -OH, -NH₂, and -N=O if H is CH; or
c) =O if H is C;
A is selected from the group consisting of CH₂, O, and C=O;
B is selected from the group consisting of CH₂, O, and -NH-;
C is selected from the group consisting of CH, and N,
D is selected from the group consisting of CH₂, O, and -NH-;
E is selected from the group consisting of CH₂, CH, and C;
F is selected from the group consisting of NH, and CH₂;
G is selected from the group consisting of NH, and CH
H is selected from the group consisting of CH₂, CH, and C;
I is selected from the group consisting of CH or CH₂;
J is selected from the group consisting of CH or CH₂;
or a pharmaceutically acceptable salt, solvate or hydrate thereof.

2. The compound of claim 1, wherein the halogen is selected from Cl, Br, I, F preferably F.

3. The compound of claim 1 or 2, wherein
R₁ is selected from the group consisting of -NH₂, -P(OH)₂, preferably -NH₂;
R₂ is selected from the group consisting of -NH₂, -P(OH)₂, preferably -NH₂;
R₃ is selected from the group consisting of -CH(OH)₂, -P(OH)₂, preferably -CH(OH)₂;
R₄ is selected from the group consisting of -P(OH)₂, -CH(OH)₂, preferably -CH(OH)₂;
A is O;
B is selected from the group consisting of CH₂, and -NH-;
C is CH;
D is selected from the group consisting of CH₂, and -NH-;
E is CH;
F is CH₂;
G is CH₂;
H is CH;
I is selected from the group consisting of CH;
J is selected from the group consisting of CH.

4. The compound of claim 1, wherein the compound is selected from the group consisting of 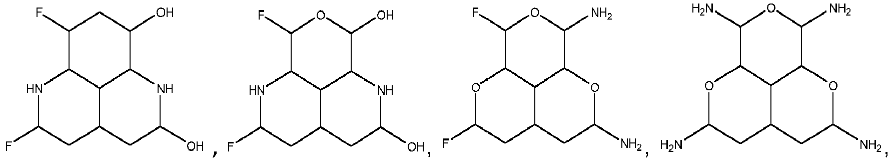 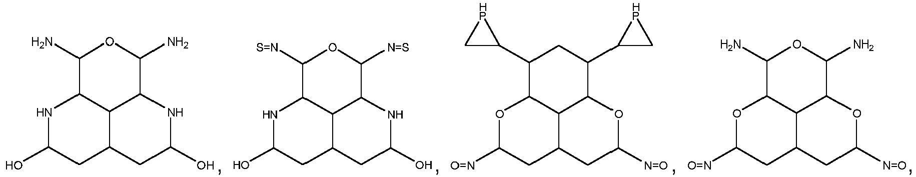 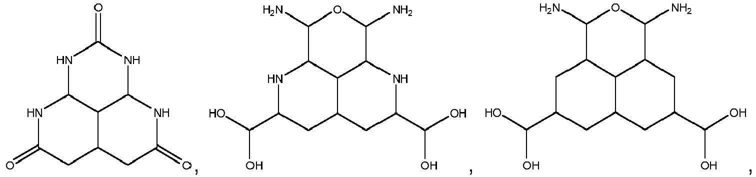 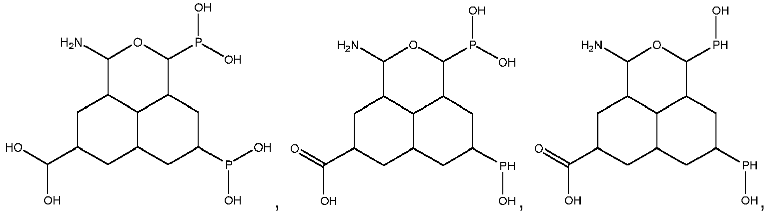 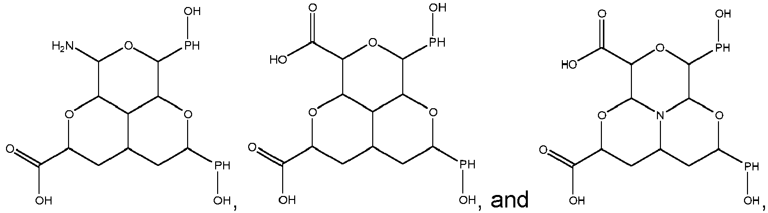 preferably 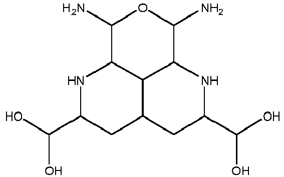 or a pharmaceutically acceptable salt, solvate or hydrate thereof.

5. The compound of claim 1, wherein the compound is selected from the group consisting of 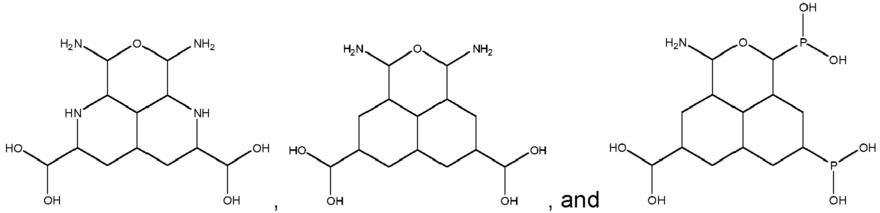 or a pharmaceutically acceptable salt, solvate or hydrate thereof.

6. The compound according to claims 1 to 5 for use in medicine.

7. A pharmaceutical composition comprising the compound according to any one of claims 1 to 5 and at leat one pharmaceutically acceptable excipient.

8. The compound of any one of claims 1 to 5 or the pharmaceutical composition of claim 6 for use in the prevention of the aggregation of phosphorylated Tau proteins.

9. The compound of any one of claims 1 to 5 or the pharmaceutical composition of claim76 for use in the treatment or prevention of a tauopathy.

10. The compound of any one of claim 1 to 5 or the pharmaceutical composition of claim 7 for use in the treatment or prevention of a neurodegenerative disease.

11. The compound or the pharmaceutical composition of claim 10, wherein the neurodegenerative disease is selected from the group consisting of Primary age-related tauopathy (PART) dementia, Chronic traumatic encephalopathy, Progressive supranuclear palsy, Corticobasal degeneration and Alzheimer's disease, preferably Alzheimer's disease.
